# EUROPEAN PATENT SPECIFICATION

(11) **EP 0 662 940 B1**
(45) Date of publication and mention of the grant of the patent: **22.10.1997**
(21) Application number: 93920059.8
(22) Date of filing: 13.08.1993
(51) Int. Cl.: C07C 17/00, C07C 1/26

(54) **PROCESS FOR CONVERTING 1,1,2-TRICHLOROETHANE TO VINYL CHLORIDE AND/OR ETHYLENE**
VERFAHREN ZUR UMWANDLUNG VON 1,1,2-TRICHLORETHAN IN VINYLCHLORID UND/ODER ETHYLEN
PROCEDE DE CONVERSION DE 1,1,2-TRICHLOROETHANE EN ETHYLENE ET/OU CHLORURE DE VINYLE

(30) Priority: 01.10.1992 US 955173
(43) Date of publication of application: 19.07.1995
(73) Proprietor: THE DOW CHEMICAL COMPANY, Midland, Michigan 48640 (US)
(72) Inventor: ITO, Larry, N., Midland, MI 48640 (US); HARLEY, A., Dale, Baton Rouge, LA 70816 (US); HOLBROOK, Michael, T., Baton Rouge, LA 70808 (US); SMITH, David, D., Baton Rouge, LA 70809 (US); MURCHISON, Craig, B., Midland, MI 48640 (US); CISNEROS, Mark, D., Baton Rouge, LA 70810 (US)
(74) Representative: Burford, Anthony Frederick
(86) International application number: US9307656
(87) International publication number: WO9407823

(56) References cited:
- EP-A- 0 015 665
- FR-A- 2 409 974

## Description

1,1,2-Trichloroethane is produced as a byproduct in the manufacture of 1,2-dichloroethane or EDC, but has limited commercial value in and of itself. The conversion of 1,1,2-trichloroethane to more useful and/or saleable, less chlorinated materials such as vinyl chloride and ethylene would be desirable, and the present invention accordingly provides a process for the accomplishment of this end.

EP-A-0496446, published on July 29, 1992, describes the preparation of chlorotrifluoroethylene and trifluoroethylene from 1,1,2-trichloro-1,2,2-trifluoroethane via a catalyst comprised of copper and a Group VIII metal (palladium and platinum being preferred) on a carbon support. Several prior publications address the same conversion and describe the same or different catalysts, see, for example EP-B-0253410, EP-B-0355907 and EP-A-0459463, all of these references, however, being directed specifically and exclusively to the chlorofluorocarbon art.

Outside of the chlorofluorocarbon art, EP-A-0015665 describes the conversion of 1,1,2-trichloroethane to ethylene and vinyl chloride via a catalyst including a noble metal chloride, an alkali metal chloride, iron chloride and optionally copper chloride.

The present invention accomplishes the catalytic conversion of 1,1,2-trichloroethane to reaction products including one or both of vinyl chloride and ethylene in commercially substantial proportions (that is, at yields (defined as the product of the conversion rate of 1,1,2-trichloroethane and the selectivity to vinyl chloride or ethylene as the case may be, on a hydrogen chloride- and hydrogen-free basis) of at least 10 percent, but preferably at least 20 percent, and more preferably at least 30 percent), by reacting 1,1,2-trichloroethane with hydrogen in the presence of a catalyst consisting of one or more Group IB metals in elemental or compound form with one or more Group VIII metals in elemental or compound form on a support.

Preferably platinum in elemental or compound form will be employed in these catalysts as a Group VIII metal, with copper in elemental or compound form being employed as a Group IB metal. More preferably, the Group IB and Group VIII metals will consist of platinum and copper in their elemental or compound forms. Especially preferred is a supported bimetallic catalyst of copper and platinum.

In general terms, a Group IB metal (copper) can be present in from 0.01 to 20 percent by weight (on an elemental basis) of these catalysts, with a Group VIII metal (platinum) comprising from 0.01 to 5.0 percent by weight (also on an elemental basis) of the catalyst. More preferably, copper will be from 0.05 to 15 percent by weight of the catalyst (on an elemental basis) and platinum will be from 0.03 to 3.0 percent by weight of the catalyst. Most preferably, the copper can be from 0.1 to 10 percent by weight of the catalyst (on an elemental basis) and platinum will be from 0.05 to 1.0 percent by weight of the catalyst.

The support can be any support which is conventionally employed in the art, but is preferably silica or carbon, with carbon being more preferred. Especially preferred is a high surface area carbon support, for example, a carbon having a specific surface area in an unimpregnated condition of 200 m²/g or more, especially 400 m²/g or more, and most especially 600 m²/g or more. An example of a commercially-available carbon which has been found suitable for use in the present invention is a coal-based carbon produced by Calgon Carbon Corporation under the designation "BPLF3", and may generally be characterized as having a specific surface area of 1100 m²/g to 1300 m²/g, a pore volume of 0.7 to 0.85 cm³/g, and an average pore radius of 12.3 to 14 angstroms (1.23 to 1.4 nm). Based on an X-ray fluorescence analysis of this carbon, a typical bulk composition of the BPLF3 carbon has been determined to be as follows (by weight percent): silicon, 1.5 percent; aluminum, 1.4 percent; sulfur, 0.75 percent; iron, 0.48 percent; calcium, 0.17 percent; potassium, 0.086 percent; titanium, 0.059 percent; magnesium, 0.051 percent; chlorine, 0.028 percent; phosphorus, 0.026 percent; vanadium, 0.010 percent; nickel, 0.0036 percent; copper, 0.0035 percent; chromium, 0.0028 percent; and manganese, 0.0018 percent (the remainder being carbon).

Process conditions can vary, depending for example on whether the process is to be conducted in the gas phase or in the liquid phase. In general, for a gas phase process, reaction pressures can range from atmospheric pressure up to 1500 psig (10.3 MPa (gauge)), with temperatures of from 100 deg. C. to 350 deg. C., residence times of from 0.25 seconds to 180 seconds, and hydrogen to 1,1,2-trichloroethane feed ratios ranging on a molar basis from 0.1:1 to 100:1. More preferably, reaction pressures will range from 5 psig (0.03 MPa (gauge)) to 500 psig (3.4 MPa (gauge)), with temperatures of from 180 deg. C. to 300 deg. C., residence times of from 0.5 seconds to 120 seconds, and hydrogen to 1,1,2-trichloroethane feed ratios of from 0.5:1 to 20:1. Most preferably, reaction pressures will range from 40 psig (0.28 MPa (gauge)) to 300 psig (2.1 MPa (gauge)), with temperatures of from 200 deg. C. to 260 deg. C., residence times of from 1 second to 90 seconds, and hydrogen to 1,1,2-trichloroethane molar feed ratios of from 0.75:1 to 6:1. For a liquid phase process, it is anticipated that reaction pressures can range from atmospheric pressure up to 3000 psig (20.6 MPa (gauge)), at temperatures from 25 to 350 degrees Celsius, residence times from one to 30 minutes, and hydrogen to 1,1,2-trichloroethane molar feed ratios of from 0.1:1 to 100:1.

Preferably hydrogen chloride will be included in the feed to the process to reduce coking and catalyst deactivation rates. In this regard, the rate of conversion loss will preferably be no more than 0.03 percent per hour, and especially no more than 0.01 percent per hour.

It is presently believed that the mechanism of the present inventive process involves the conversion of 1,1,2-trichloroethane to vinyl chloride, and the subsequent conversion of vinyl chloride to ethylene. It should be well within the skill of the art, given this proposed mechanism and the examples which follow, to tailor the process of the present invention to produce a desired product or a desired proportion of products.

### Illustrative Examples

### Example 1

A catalyst was initially prepared containing 0.5 percent by weight of platinum and 0.9 percent by weight of copper on Calgon's BPLF3 carbon support. An aqueous H₂PtCl₆ stock solution was first prepared by dissolving H₂PtCl₆·6H₂O (J. T. Baker, Inc.; Baker Analyzed Grade, 37.6 percent Pt) in deionized and distilled water. An amount of CuCl₂ (Aldrich Chemical Company, Inc., 99.999 percent purity) was placed in a 250 mL Erlenmeyer flask, and the H₂PtCl₆ stock solution added in an appropriate proportion with swirling to dissolve the CuCl₂. The solution was then diluted with deionized, distilled water and swirled. Calgon BPLF3 activated carbon (6 x 16 mesh (3.4 x 1.2 mm), Calgon Carbon Corp., Pittsburgh, Pa.) was added to the flask, and the flask was agitated rapidly so that the carbon carrier was evenly coated with the aqueous Pt/Cu solution. The catalyst preparation was dried in an evaporating dish in air at ambient temperatures for 18 hours, and then further dried in an oven in air at 120 degrees Celsius for 2 hours.

A catalyst charge (0.6 grams) of the thus-dried catalyst was placed in a tubular reactor within the oven compartment of a gas chromatograph (as described below), over a glass wool support contained in the center of the reactor tubing. The catalyst was then covered with a plug of glass wool.

The charged catalyst was dried for from 8 to 24 hours at 150 degrees Celsius under a nitrogen purge, and thereafter reduced by passing hydrogen through the reactor at a flow rate of 34 mL/minute for 24 hours. The reactor temperature was then adjusted to the desired temperature setpoint of 235 degrees Celsius (at 76 psig (0.52 MPa (gauge))). The reactor temperature and hydrogen gas flow were allowed to equilibrate for about 1 hour before a liquid 1,1,2-trichloroethane flow was started.

The 1,1,2-trichloroethane was pumped via a high pressure syringe pump through 1/16th inch (1.6 mm) (O.D.) Monel™ nickel alloy tubing (unless specifically noted below all of the components, tubing and fittings of the test reactor apparatus were also made of Monel™ nickel alloy (Huntington Alloys, Inco Alloys International, Inc.)) into a packed sample cylinder serving as a feed evaporator.

The 1/16th inch (1.6 mm) tubing extended almost to the center of the packed cylinder, which was heated to a vaporizing temperature of 180 degrees Celsius using electrical heat tracing. Vaporization of the 1,1,2-trichloroethane was accomplished in the feed line, so that the 1,1,2-trichloroethane was superheated when combined with the hydrogen feed stream. Thermocouples were used to monitor the skin temperature of the feed evaporator and the temperature of the gas exiting the feed evaporator.

The hydrogen feed stream was metered (at a 1:1 molar ratio with the 1,1,2-trichloroethane) to a preheater using a Model 8249 linear mass flow controller from Matheson Gas Products, Inc. Secaucus, N.J., with the preheater consisting of a packed sample cylinder wrapped with electrical heat tracing. Thermocouples were used to monitor both the skin temperature of the preheater and the temperature of the gas exiting the preheater. The preheater temperature was set and maintained at 140 degrees Celsius.

Vaporized 1,1,2-trichloroethane exiting the evaporator was mixed with the hydrogen gas from the preheater in a 2 foot (0.61 meters) long section of 1/4 inch (0.64 cm) tubing maintained at a temperature of 140 degrees Celsius. The mixed gases then were passed into and reacted within a tubular reactor (1/2 inch (1.27 cm) O.D., 12 inches (30.5 cm) in length) located within an aluminum block equipped with a cartridge heater to achieve a desired reaction temperature, which in this case was 235 degrees Celsius. The residence time was 5.0 seconds.

After thus reacting the 1,1,2-trichloroethane and hydrogen, the products from the reaction were passed to a gas sampling valve, which provided gaseous aliquots for online gas chromatographic analysis in a Hewlett-Packard Model 5890 Series II gas chromatograph (Hewlett-Packard Company). The gas chromatograph was equipped with a flame ionization detector, and used 30 meter by 0.53 millimeter (I.D.) 100 percent methyl silicone/fused silica and 30 meter by 0.53 millimeter (I.D.) porous polymer-lined fused silica columns to separate the various reaction products. Response factors were conventionally determined by injections of gravimetrically-prepared standards of the individual reaction products. These response factors were applied in conjunction with individual peak areas and the total mols of all reaction products to determine the mol percents of individual components in the reactor effluent, and the selectivity to individual reaction products.

Eighty (80) percent of the 1,1,2-trichloroethane was shown by this analysis to have been converted to reaction products including vinyl chloride at 76 percent selectivity (for a vinyl yield of 60.8 percent ((80 x 76)/100)) , ethylene at 14 percent selectivity (for an ethylene yield of 11.2 percent) and ethane at 8 percent selectivity.

### Example 2

The catalyst, apparatus and procedures of Example 1 were employed for this Example, except the ratio of hydrogen to 1,1,2-trichloroethane was changed to 3:1.

Eighty-four (84) percent of the 1,1,2-trichloroethane was converted, with vinyl being produced at 86 percent selectivity, ethylene at 8 percent selectivity, and ethane at 5.5 percent selectivity.

### Example 3

The apparatus and procedures of the previous examples were employed in this Example as well, but with a different catalyst and under different reaction conditions.

The catalyst was a bimetallic platinum/copper catalyst on the same carbon as in Examples 1 and 2, except platinum was 0.25 percent by weight of the catalyst and copper was 0.50 percent by weight of the catalyst. The hydrogen and 1,1,2-trichloroethane were fed to the reactor at a molar ratio of 5.0:1, at 250 degrees Celsius and with a residence time of 2.1 seconds. Under these conditions, 99 percent of the 1,1,2-trichloroethane was converted to reaction products including ethylene at 44 percent selectivity (for a yield of 43.6 percent), vinyl chloride at 40 percent selectivity (for a yield of 39.6 percent) and ethane at 15 percent selectivity.

While various embodiments of the processes and catalysts of the present invention have been described and/or exemplified herein, those skilled in the art will readily appreciate that numerous changes can be made thereto which are nevertheless properly considered to be within the scope of the present invention as defined by the claims below.

## Claims

1. A process for the conversion of 1,1,2-trichloroethane to reaction products including one or both of vinylidene chloride and ethylene by reaction with hydrogen in the presence of a supported catalyst characterized in that the catalyst consists of one or more Group IB metals in elemental or compound form and one or more Group VIII metals in elemental or compound form on the support.

2. A process as claimed in Claim 1, wherein hydrogen chloride is incorporated in the feed to the process.

3. A process as claimed in Claim 1 or Claim 2, wherein the one or more Group IB metals includes copper and wherein the one or more Group VIII metals includes platinum.

4. A process as claimed in Claim 3, wherein the Group IB and Group VIII metals consist of copper and platinum.

5. A process as claimed in any one of the preceding claims, wherein the catalyst comprises from 0.01 to 5.0 percent by weight of Group VIII metal or compound on an elemental basis and from 0.01 to 20 percent by weight of Group IB metal or compound, also on an elemental basis.

6. A process as claimed in Claim 5, wherein the catalyst comprises from 0.03 to 3.0 percent by weight of platinum on an elemental basis and from 0.05 to 15 percent by weight of copper, also on an elemental basis.

7. A process as claimed in Claim 6, wherein the catalyst comprises from 0.05 to 1.0 percent by weight of platinum on an elemental basis and from 0.1 to 10 percent by weight of copper, also on an elemental basis.

8. A process as claimed in any one of the preceding claims, wherein the catalyst support is a carbon having a specific surface area of at least 200 m²/g.

9. A process as claimed in Claim 8, wherein the catalyst support is a carbon having a specific surface area of at least 400 m²/g.

10. A process as claimed in Claim 9, wherein the catalyst support is a carbon having a specific surface area of at least 600 m²/g.

11. A process as claimed in any one of the preceding claims, wherein the reaction is conducted in the gas phase at a pressure of from atmospheric pressure to 10.3 MPa (gauge), at a temperature of from 100 degrees Celsius to 350 degrees Celsius, a residence time of from 0.25 seconds to 180 seconds, and a hydrogen to 1,1,2-trichloroethane molar feed ratio of from 0.1:1 to 100:1.

12. A process as claimed in Claim 11, wherein the reaction is conducted in the gas phase at a pressure of from 0.03 MPa (gauge) to 3.4 MPa (gauge), at a temperature of from 180 degrees Celsius to 300 degrees Celsius, a residence time of from 0.5 seconds to 120 seconds, and a hydrogen to 1,1,2-trichloroethane molar feed ratio of from 0.5:1 to 20:1.

13. A process as claimed in Claim 12, wherein the reaction is conducted in the gas phase at a pressure of from 0.28 MPa (gauge) to 2.1 MPa (gauge), at a temperature of from 200 degrees Celsius to 260 degrees Celsius, a residence time of from 1 second to 90 seconds, and a hydrogen to 1,1,2-trichloroethane molar feed ratio of from 0.75:1 to 6:1.

## Patentansprüche

1. Verfahren zur Überführung von 1,1,2-Trichlorethan in Reaktionsprodukte, die Vinylidenchlorid oder/und Ethylen enthalten, durch Reaktion mit Wasserstoff in der Gegenwart eines Katalysators auf einem Träger, dadurch gekennzeichnet, daß der Katalysator aus einem oder mehreren Gruppe IB Metallen in elementarer Form oder Verbindungsform und einem oder mehreren Gruppe VIII Metallen in elementarer Form oder Verbindungsform auf dem Träger besteht.

2. Verfahren nach Anspruch 1, wobei der Zufuhr zu dem Verfahren Chlorwasserstoff zugesetzt wird.

3. Verfahren nach Anspruch 1 oder Anspruch 2, wobei das oder die Gruppe IB Metalle Kupfer umfassen und das oder die Gruppe VIII Metalle Platin umfassen.

4. Verfahren nach Anspruch 3, wobei die Gruppe IB und Gruppe VIII Metalle aus Kupfer und Platin bestehen.

5. Verfahren nach einem der vorhergehenden Ansprüche, wobei der Katalysator von 0,01 bis 5,0 Gew.-% Gruppe VIII Metall oder Verbindung auf elementarer Basis und von 0,01 bis 20 Gew.-% Gruppe IB Metall oder Verbindung, ebenfalls auf elementarer Basis, umfaßt.

6. Verfahren nach Anspruch 5, wobei der Katalysator von 0,03 bis 3,0 Gew.-% Platin auf elementarer Basis und von 0,05 bis 15 Gew.-% Kupfer, ebenfalls auf elementarer Basis, umfaßt.

7. Verfahren nach Anspruch 6, wobei der Katalysator von 0,05 bis 1,0 Gew.-% Platin auf elementarer Basis und von 0,1 bis 10 Gew.-% Kupfer, ebenfalls auf elementarer Basis, umfaßt.

8. Verfahren nach einem der vorhergehenden Ansprüche, wobei der Katalysator-Träger ein Kohlenstoff mit einer spezifischen Oberfläche von mindestens 200 m²/g ist.

9. Verfahren nach Anspruch 8, wobei der Katalysator-Träger ein Kohlenstoff mit einer spezifischen Oberfläche von mindestens 400 m²/g ist.

10. Verfahren nach Anspruch 9, wobei der Katalysator-Träger ein Kohlenstoff mit einer spezifischen Oberfläche von mindestens 600 m²/g ist.

11. Verfahren nach einem der vorhergehenden Ansprüche, wobei die Reaktion in der Gasphase bei einem Druck von Atmosphärendruck bis 10,3 MPa (Überdruck), bei einer Temperatur von 100 Grad Celsius bis 350 Grad Celsius, einer Verweilzeit von 0,25 Sekunden bis 180 Sekunden und einem molaren Zufuhrverhältnis von Wasserstoff zu 1,1,2-Trichlorethan von 0,1:1 bis 100:1 durchgeführt wird.

12. Verfahren nach Anspruch 11, wobei die Reaktion in der Gasphase bei einem Druck von 0,03 MPa (Überdruck) bis 3,4 MPa (Überdruck), bei einer Temperatur von 180 Grad Celsius bis 300 Grad Celsius, einer Verweilzeit von 0,5 Sekunden bis 120 Sekunden und einem molaren Zufuhrverhältnis von Wasserstoff zu 1,1,2-Trichlorethan von 0,5:1 bis 20:1 durchgeführt wird.

13. Verfahren nach Anspruch 12, wobei die Reaktion in der Gasphase bei einem Druck von 0,28 MPa (Überdruck) bis 2,1 MPa (Überdruck), bei einer Temperatur von 200 Grad Celsius bis 260 Grad Celsius, einer Verweilzeit von 1 Sekunde bis 90 Sekunden und einem molaren Zufuhrverhältnis von Wasserstoff zu 1,1,2-Trichlorethan von 0,75:1 bis 6:1 durchgeführt wird.

## Revendications

1. Procédé pour la conversion du 1,1,2-trichloro-éthane en produits de réaction comprenant du chlorure de vinylidène, de l'éthylène ou les deux à l'aide d'une réaction avec de l'hydrogène en présence d'un catalyseur sur support, caractérisé en ce que le catalyseur est constitué d'un ou de plusieurs métaux du groupe IB sous forme élémentaire ou de composé et d'un ou de plusieurs métaux du groupe VIII sous forme élémentaire ou de composé sur le support.

2. Procédé selon la revendication 1, dans lequel du chlorure d'hydrogène est incorporé dans l'alimentation du procédé.

3. Procédé selon l'une des revendications 1 ou 2, dans lequel le (ou les) métal (métaux) du groupe IB comprend (comprennent) le cuivre et dans lequel le (ou les) metal (métaux) du groupe VIII comprend (comprennent) le platine.

4. Procédé selon la revendication 3, dans lequel les métaux du groupe IB et du groupe VIII sont le cuivre et le platine.

5. Procédé selon l'une des revendications précédentes, dans lequel le catalyseur comprend de 0,01 à 5,0 % en poids, sur une base élémentaire, du métal ou du composé du métal du groupe VIII, de 0,01 à 20 % en poids, également sur une base élémentaire, du métal ou du composé du métal du groupe IB.

6. Procédé selon la revendication 5, dans lequel le catalyseur se compose de 0,03 à 3,0 % en poids de platine sur une base élémentaire et de 0,05 à 15 % en poids de cuivre également sur une base élémentaire.

7. Procédé selon la revendication 6, dans lequel le catalyseur se compose de 0,05 à 1,0 % en poids de platine sur une base élémentaire et de 0,1 à 10 % en poids de cuivre également sur une base élémentaire.

8. Procédé selon l'une des revendications précédentes, dans lequel le support de catalyseur est un carbone ayant une surface spécifique d'au moins 200 m²/g.

9. Procédé selon la revendication 8, dans lequel le support de catalyseur est un carbone ayant une surface spécifique d'au moins 400 m²/g.

10. Procédé selon la revendication 9, dans lequel le support de catalyseur est un carbone ayant une surface spécifique d'au moins 600 m²/g.

11. Procédé selon l'une des revendications précédentes, dans lequel la réaction est effectuée en phase gazeuse sous une pression allant de la pression atmosphérique à 10,3 MPa (au manomètre), à une température de 100 degrés Celsius à 350 degrés Celsius, pendant une durée de séjour de 0,25 seconde à 180 secondes, et avec un rapport molaire de l'hydrogène au 1,1,2-trichloroéthane de 0,1:1 à 100:1 dans l'alimentation.

12. Procédé selon la revendication 11, dans lequel la réaction est effectuée en phase gazeuse sous une pression de 0,03 MPa (au manomètre) à 3,4 MPa (au manomètre), à une température de 180 degrés Celsius à 300 degrés Celsius, pendant une durée de séjour de 0,5 seconde à 120 secondes, et avec un rapport molaire de l'hydrogène au 1,1,2-trichloroéthane de 0,5:1 à 20:1 dans l'alimentation.

13. Procédé selon la revendication 12, dans lequel la réaction est effectuée en phase gazeuse sous une pression de 0,28 MPa (au manomètre) à 2,1 MPa (au manomètre), à une température de 200 degrés Celsius à 260 degrés Celsius, pendant une durée de séjour de 1 seconde à 90 secondes, et avec un rapport molaire de l'hydrogène au 1,1,2-trichloroéthane de 0,75:1 à 6:1 dans l'alimentation.
